**Europäisches Patentamt**

**European Patent Office**

**Office européen des brevets**

(19)

(11) Veröffentlichungsnummer : **0 423 530 B1**

(12) # EUROPÄISCHE PATENTSCHRIFT

(45) Veröffentlichungstag der Patentschrift :
**05.01.94 Patentblatt 94/01**

(51) Int. Cl.$^5$ : **C01B 35/12, B01J 29/04**

(21) Anmeldenummer : **90118806.0**

(22) Anmeldetag : **01.10.90**

(54) **Verfahren zur Herstellung synthetischer, kristalliner, metallhaltiger Borosilikate.**

(30) Priorität : **16.10.89 DD 333613**

(43) Veröffentlichungstag der Anmeldung :
**24.04.91 Patentblatt 91/17**

(45) Bekanntmachung des Hinweises auf die
Patenterteilung :
**05.01.94 Patentblatt 94/01**

(84) Benannte Vertragsstaaten :
**AT BE CH DE DK ES FR GB GR IT LI LU NL SE**

(56) Entgegenhaltungen :
**EP-A- 0 091 508**
**DD-A- 207 186**
**US-A- 3 328 119**
**US-A- 4 285 919**

(73) Patentinhaber : **VAW Aluminium AG**
**Georg-von-Boeselager-Strasse 25**
**D-53117 Bonn (DE)**
Patentinhaber : **LEUNA-WERKE AG**
**Am Haupttor**
**D-06236 Leuna (DE)**

(72) Erfinder : **Unger, Baldur, Dr.**
**Saalfelder Strasse 130**
**O-6421 Reichmannsdorf (DE)**
Erfinder : **Becker, Karl, Dr.**
**August-Bebel-Strasse 2**
**W-4807 Bad Kösen (DE)**
Erfinder : **Thome, Roland, Dr.**
**Brüsseler Strasse 58**
**W-5300 Bonn 1 (DE)**
Erfinder : **Tissler, Arno, Dr.**
**Koernicke Strasse 2**
**W-5300 Bonn 1 (DE)**
Erfinder : **Schwieger, Wilhelm, Dr.**
**Zwingerstrasse 10**
**W-04020 Halle (DE)**
Erfinder : **Tschritter, Hartmut, Dr.**
**An der Kastanie 3**
**W-06500 Gera (DE)**

(74) Vertreter : **Müller-Wolff, Thomas**
**HARWARDT NEUMANN, Patent- und**
**Rechtsanwälte, Postfach 14 55**
**D-53704 Siegburg (DE)**

**EP 0 423 530 B1**

## Beschreibung

Verfahren zur Herstellung synthetischer, kristalliner, metallhaltiger Borosilikate, einem danach hergestellten Borosilikat sowie einem unter Verwendung des Borosilikats hergestelltem Katalysator.

Die Erfindung betrifft Verfahren zur Herstellung synthetischer, kristalliner, metallhaltiger Borosilikate mit Pentasilstruktur in einem Kristallisationsprozeß aus einer Reaktionsmischung, die Verbindung von Silicium, Bor, Natrium, Hydrooxidionen sowie Wasser, jedoch keine Struktur dirigierenden organischen Verbindungen oder Stickstoffverbindungen enthält, gekennzeichnet dadurch, daß der Reaktionsmischung mindestens eine Aluminiumverbindung und mindestens ein Nebengruppenelement der 4. Periode des Periodensystems zugesetzt wird sowie ein danach hergestelltes Borosilikat und einen unter Verwendung des Borosilikats hergestellten Katalysators.

Zeolithe mit Pentasilstruktur, als deren bekannteste Vertreter der ZSM-5 und der ZSM-11 gelten, werden als wirksame Katalysatoren bzw. Katalysatorkomponenenten für die Umwandlung von Kohlenwasserstoffen sowie von sauerstoff- und anderen heteroatomfunktionalisierten organischen Verbindungen beschrieben. Es ist bekannt, daß derartige Zeolithe für die Umwandlung von Methanol oder Dimethylether zu Kohlenwasserstoffen (z.B. US-PS 3.333.250), zur spaltenden Bearbeitung von höhersiedenden Kohlenwasserstofffraktionen (US-PS 3.758.403), zur Isomerisierung von $C_8$-Aromaten (z.B. US-PS 4.224.141), zur paraselektiven Alkylierung oder Disproportionierung von Toluen (US-PS 4.097.543, US-PS 4.052.476) sowie für viele andere spezielle Reaktionen angewandt werden können. Eine Besonderheit der Pentasilzeolithe besteht darin, daß sie über eine große Variabilität in der Zusammensetzung verfügen und demnach praktisch ein für jede Reaktion optimiertes pentasilhaltiges Katalysatorsystem denkbar ist.

Bekannt sind beispielsweise Verfahren, bei denen eine Verbesserung insbesondere der katalytischen Eigenschaften entsprechender Zeolithe durch die Zugabe von Substanzen, die Elemente enthalten, welche als potentielle Substituenten des Siliziums im Zeolithgitter in Frage kommen, zur Synthesemischung erreicht wurde (US-PS 4.269.813, US-PS 4.337.176). Dabei konnte nachgewiesen werden, daß bei borhaltigen Pentasilizeolithen das Bor praktisch vollständig isomorph in das Gerüst eingebaut wird. Dies führt in vielen Fällen zu einer Verbesserung der Katalysatoreigenschaften. Die Herstellung von Pentasilzeolithen mit definierter Zusammensetzung läßt sich in bisher bekannter Weise realisieren, wenn man den zur hydrothermalen Kristallisation verwendeten Silikathydrogelen eine oder mehrere strukturdirigierende organische Substanzen, sogenannte Template-Verbindungen, wie z.B. quarternäre Alkylammoniumsalze, Amine, Alkohole u.a. zusetzt (US-PS 3.702.886, US-PS 3.941.871, US-PS 4.112.056, DE-OS 2.442.240, DE-OS 2.817.576, DE-OS 2.913.552). Gleiches gilt auch für Borosilikate mit Pentasilstruktur (BE 880.858, DE-OS 3.316.488, US-P 4.285.919, DE-OS 3.143.045, EP 113.473). Nachteilig bei diesen Synthesevarianten unter Zusatz von Template-Verbindungen ist jedoch der hohe technische und ökonomische Aufwand zur Rückgewinnung nicht umgesetzten Templates sowie das unumgängliche Abbrennen von im Zeolithgefüge eingebauten organischen Substanzen, das einerseits einen hohen Aufwand zur Entsorgung erfordert, andererseits zu mitunter erheblichen irreversiblen Schädigungen am Zeolith selbst führt. Als Alternative dazu sind Verfahren zur Kristallisation von Pentasilzeolithen bekannt, die auf den Einsatz organischer Template-Verbindungen verzichten (DE-OS 3.242.352, DD-PS 207.186). Der Nachteil dieser Verfahren besteht jedoch darin, daß die Variabilität in der Zusammensetzung der Kristallisationsprodukte gegenüber den Template-haltigen Reaktionsmischungen stark eingeschränkt wird.

Verfahren zur Herstellung von Borosilikaten mit Zeolithstruktur, einschließlich solcher, die Metalle enthalten, aus einer von organischen Verbindungen freien Reaktionsmischung sind nicht bekannt.

Ein Verfahren zur Herstellung synthetischer, kristalliner, metallhaltiger Borosilikate aus einer Reaktionsmischung, die Verbindungen von Silizium, Bor, Natrium, Hydroxidionen sowie Wasser ohne Zusatz organischer oder stickstoffenthaltender Verbindungen enthält, ist in der US-PS 3.328.119 beschrieben. Bei der Herstellung des bekannten Borosilikats wird eine Aluminiumverbindung in Form von alkaliluminat, aluminatgel und aluminasole zu der Reaktionsmischung zugesetzt wird. Die Reaktionsmischung wird vor der Kristallisation über einen Zeitraum von 1 bis 10 Stunden bei Raumtemperatur gealtert und danach ein Festkörper in "klassischer" Zeolithstruktur als Faujasite, Mordenit oder Zeolitha erhalten.

Für die Verwendung derartiger Zeolithe als Katalysatorkomponente ist es notwendig, neben der ursprünglich vorhandenen Säurefunktion weitere katalytisch aktive Funktionen in Form von Hydrier- , Dehydrier- oder Oxidationfunktionen in das Zeolithgefüge einzubringen. Dies erfolgt in nachgeordneten Verfahrensschritten, wie z. B. einen Ionenaustausch, einer Imprägnierung oder eines mechanischen Vermischens, wobei die Metallkomponente in die Poren bzw. Zwischenräume des kristallinen Produkts nachträglich eingebracht wird.

Derartige nachgeordnete Verfahrensschritte sind aus der US-PS 4.285.919 auch für borhaltige Pentasilzeolithtypen bekannt, die aus templatehaltigen Reaktionsmischungen erhalten wurden. Eine aus erfahrungstechnischen Gründen günstige Einbringung der Metallkomponente bereits im Prozeß der Zeolithsynthese ist bisher nicht durchgeführt worden, da die in Betracht kommenden Elemente dem Kristallisationverlauf beeinträchtigen können. Daher ist auch die Herstellung metallhaltiger Borosilikate mit Pentasilstruktur auf direkt synthetischem Wege ohne Zusatz von strukturdirigierenden, organischen oder Stickstoffverbindungen bisher nicht bekannt.

Der Erfindung liegt die Aufgabe zugrunde, ein Verfahren zur Herstellung synthetischer, kristalliner, metallhaltiger Borosilikate mit Pentasilstruktur zu schaffen, welches auf die Verwendung organischer Verbindungen oder Stickstoffverbindungen verzichtet sowie kostengünstig und umweltfreundlich betrieben werden kann. Ferner soll ein unter Verwendung des Verfahrens hergestelltes Borosilikat sowie ein das Borosilikat enthaltender Katalysator angegeben werden.

Die Aufgabe wird erfindungsgemäß durch die im Hauptanspruch angegebenen Merkmale gelöst, wobei ein erfindungsgemäß hergestelltes Borosilikat im Anspruch 16 und ein unter Verwendung des Borosilikats hergestellter Katalysator im Anspruch 17 angegeben ist.

Die Aufgabe, ein Verfahren zur Herstellung synthetischer, kristalliner, metallhaltiger Borosilikate zu schaffen, wird im wesentlichen damit gelöst, daß eine Reaktionsmischung, die Verbindung von Silizium, Bor, Natrium und mindestens einem Nebengruppenelement der 4. Periode des Periodensystems der Elemente, insbesondere Chromium, Nikkel, Kobalt, Eisen, Gallium, Zink oder Mangan, neben Hydroxidionen und Wasser, jedoch keine als Template wirkende organische Verbindung enthält, unter Zusatz von mindestens einer Aluminiumverbindung, bei Einhaltung eines molaren Verhältnisses B/(B+Al) von 0,01 bis 1 sowie eines molaren Verhältnisses M/(B+Al) von 0,05 bis 5 über einen Zeitraum von 1 bis 100 Stunden, vorzugsweise 5 bis 20 Stunden unter hydrothermalen Bedingungen bei 403 K bis 593 K einem Kristallisationsprozeß unterworfen wird.

M bedeutet Nebengruppenelement der 4. Periode des Periodensystems der Elemente.

Überraschend war, daß auf diese Weise ohne Zusatz von organischen Substanzen oder Stickstoffverbindungen synthetische, kristalline, metallhaltige Borosilikate mit vorteilhaften Eigenschaften sowie vorteilhaften Eigenschaften für Stofftrennung und Stoffreinigung erhalten werden. Als Quellen der zur Synthese verwendeten Elemente bzw. deren Oxide können beispielsweise dienen:

- für Natriumoxid: Natriumhydroxid oder Natriumnitrat;
- für Siliziumdioxid: pyrogene oder Fällungskieselsäuren, Kieselsäuresole, Alkalimetallsilikate und/oder Wasserglaslösungen;
- für Boroxid: Borsäure, Bortrifluorid oder Borax;
- für die genannten Nebengruppenelemente: Salz (insbesondere Nitrate), hydratisierte bzw. hydratisierbares Aluminiumoxid, Aluminiumsalze, insbesondere Aluminiumsulfat und/oder Aluminatlösungen.

Die Vermischung der Ausgangsstoffe kann diskontinuierlich in den für die Reaktion vorgesehenen Gefäßen, jedoch auch kontinuierlich in einer der Kristallisation vorgelagerten Stufe erfolgen. Typische Zusammensetzung der Reaktionsmischung, ausgedrückt in Molverhältnissen der eingesetzten Elemente bzw. deren Oxide sind:

$Na_2O/SiO_2$      0,05 bis 0,5
$H_2O/SiO_2$      5 bis 150.
$SiO_2/Al_2O_3$      $\geqq$ 10
B/(B+Al)      0,1 bis 1,0
M/(Al+B)      0,05 bis 5

Bei einer Reaktionstemperatur von 403 K bis 593 K wird diese Mischung unter hydrothermalen Bedingungen kristallisiert, das Kristallisat anschließend von der Mutterlauge getrennt, gewaschen und getrocknet. Durch 1 bis 24 stündiges Altern der Reaktionsmischung bei Raumtemperatur und/oder durch Zugabe von Kristallisationskeimen aus kristallinen oder teilkristallinen Borosilikaten gleicher oder ähnlicher Struktur kann die Kristallisation beschleunigt werden und die Einheitlichkeit des Kristallisats verbessert werden.

Die auf diese Weise erhaltenen kristallinen, metallhaltigen Borosilikate erzeugen charakteristische Röntgenbeugungslinien gemäß Tab. 1, sind frei von organischen Bestandteilen und Stickstoffverbindungen und weisen im dehydratisierten Zustand vorzugsweise folgende Zusammensetzung auf:

0,8 bis 1,5 $Na_2O$ * x $B_2O_3$ * y $Al_2O_3$ * z $M_2O$ * 10 bis 100 $SiO_2$ mit x+y=1, x//x+y)=0,1 bis 1,0 und z/(x+y)=0,05 bis 5.

Die erfindungsgemäßen Metallsilikate können für den Einsatz z.B. in Katalysatoren oder Adsorbentien durch auf dem Fachgebiet bekannte Techniken modifiziert und/oder geformt werden.

## Ausführungsbeispiele

Es bedeuten Ma.-% Masseanteile in Prozent.

**Beispiel 1 (Vergleichsbeispiel)**

In Anlehnung an DD 207.186 wird eine Lösung hergestellt aus 2,21 g Natriumhydroxid (p.a.), 243 g Wasser und 8,46 g Natriumaluminat (ca. 13 Ma.-% $Al_2O_3$). Zu dieser alkalischen Lösung werden bei Raumtemperatur unter intensivem Rühren 86,05 g Natrium-stabilisierte Kieselsol (ca. 31 Ma.-% $SiO_2$) zugefügt und homogenisiert. Die Zusammensetzung der Reaktionsmischung ergibt sich zu folgenden Molverhältnissen:

$Na_2O/SiO_2$  0,1
$H_2O/Na_2O$  300
$SiO_2/Al_2O_3$  40

Das Reaktionsgemisch wird unter Eigendruck bei 473 K zur Kristallisation gebracht. Nach einer Kristallisationszeit von 9 Stunden wird ein Produkt erhalten, das die in Tabelle 1 angeführten typischen Röntgenbeugungslinien (hier ausgedrückt in Werten der entsprechenden Netzebenenabstände) hervorruft. Die auf beschriebene Weise hergestellte Probe besitzt Vergleichscharakter. Eine elektronenmikroskopische Aufnahme des Kristallisats ist in Bild 1 dargestellt. Als einen, das Volumen der Elementarzelle beschreibenden Parameter wurde, ähnlich, wie von Meyers (B.L. Meyers et al., J. Catal. 91(1985)352) vorgestellt, die Summe der Netzebenenabstände, abgeleitet aus der Lage der 5 intensivsten Reflexe im Glanzwinkelbereich von 45 Grad bis 52 Grad (2 Theta) des Röntgendiffraktogramms der untersuchten Proben herangezogen. Bei der nach Beispiel 1 hergestellten Vergleichsprobe wird der Wert dieses Parameters zu 974,82 pm bestimmt.

Tabelle 1: Werte der charakteristischen Röntgenbeugungslinien der Vergleichsprobe entsprechend Beispiel 1

| Netzebenenabstand (nm) | | relative Intensität |
|---|---|---|
| 1,11 | +/- 0,02 | st |
| 0,980 | +/- 0,02 | st |
| 0,587 | +/- 0,01 | s |
| 0,557 | +/- 0,01 | s |
| 0,548 | +/- 0,01 | ss |
| 0,421 | +/- 0,01 | s |
| 0,386 | +/- 0,007 | sst |
| 0,381 | +/- 0,007 | st |
| 0,372 | +/- 0,005 | m |
| 0,369 | +/- 0,005 | m |
| 0,362 | +/- 0,005 | m |
| 0,340 | +/- 0,005 | s |
| 0,329 | +/- 0,003 | ss |
| 0,302 | +/- 0,003 | s |
| 0,297 | +/- 0,003 | s |
| 0,294 | +/- 0,003 | s |

**Beispiel 2**

Es wird eine Lösung, bestehend aus 4,23 g Natriumaluminat, 2,88 g Natriumhydroxid, 0,67 g Borsäure und 243 g Wasser bereitet, unter kräftigem Rühren 86,05 g Natrium-stabilisiertes Kieselsol zugesetzt und das Reaktionsgemisch intensiv homogenisiert. Die molare Zusammensetzung der Reaktionspartner ergibt sich zu:

$Na_2O/SiO_2$  0,16
$H_2O/Na_2O$  185

SiO$_2$/Al$_2$O$_3$     80
B/(B+Al)     0,5

Das Reaktionsgemisch wird unter Eigendruck bei 448 K zur Kristallisation gebracht. Nach 20 Stunden wird ein Produkt erhalten, das ein Pentasil-typisches Röntgendiffraktogramm hervorruft. Der elementarzellenvolumenproportionale Parameter nimmt einen Wert von 972,9 pm an.

**Beispiel 3**

Es wird eine Lösung, bestehend aus 4,25 g Natriumhydroxid, 0,36 g Borsäure, 2,28 g Cr(NO$_3$)$_3$ * 9 H$_2$O, 4,23 g Natriumaluminat und 243 g Wasser bereitet, unter kräftigem Rühren 86,05 g Natrium-stabilisiertes Kieselsol zugesetzt und das Reaktionsgemisch intensiv homogenisiert. Die molare Zusammensetzung der Reaktionspartner ergibt sich zu
Na$_2$O/SiO$_2$     0,16
H$_2$O/Na$_2$O     185
SiO$_2$/Al$_2$O$_3$     80
B/(B+Al)     0,5
Cr/Al     1

Das Reaktionsgemisch wird unter Eigendruck bei 448 K zur Kristallisation gebracht. Nach 20 Stunden wird ein Produkt erhalten, das ein Pentasil-typisches Röntgendiffraktogramm hervorruft. Der elementarzellenvolumenproportionale Parameter nimmt einen Wert von 973,3 pm an.

**Beispiel 4**

Es wird eine Lösung hergestellt aus 3,58 g Natriumhydroxid, 2,19 g Ni(NO$_3$)$_3$ * 6 H$_2$O, 0,44 g Borsäure, 243 g Wasser und 5,58 g Natriumaluminat, in diese Lösung 86,05 g Natrium-stabilisiertes Kieselsol eingebracht und intensiv homogenisiert. Die molare Zusammensetzung der Reaktionsmischung ergibt sich zu:
Na$_2$O/SiO$_2$     0,14
H$_2$O/Na$_2$O     212.
SiO$_2$/Al$_2$O$_3$     80
B/(B+Al)     0,5
Ni/Al     1

Das Reaktionsgemisch wird unter Eigendruck bei 453 K zur Kristallisation gebracht und nach 17 Stunden ein Produkt getrennt, das sich röntgenographisch als Pentasil-typisches Kristallisat erweist. Der das Elementarzellenvolumen charakterisierende Parameter nimmt einen Wert von 974,2 pm an.

**Beispiel 5**

Es wird eine Lösung hergestellt aus 3,79 g Natriumhydroxid, 0,67 g Borsäure, 3,32 g Co(NO$_3$)$_2$ * 6 H$_2$O, 243 g Wasser und 4,23 g Natriumaluminat, in diese 86,05 g Natrium-stabilisiertes Kieselsol eingerührt und intensiv homogenisiert. Die molare Zusammensetzung des Reaktionsgemisches beträgt:
Na$_2$O/SiO$_2$     0,14
H$_2$O/Na$_2$O     212.
SiO$_2$/Al$_2$O$_3$     80
B/(B+Al)     0,5
Co/Al     2

Die Synthesemischung wird unter Eigendruck bei 473 K zur Kristallisation gebracht. Nach 15 Stunden entsteht ein Pentasil-typisches Kristallisat. Der das Elementarzellenvolumen charakterisierende Parameter beträgt 973,9 pm.

**Anwendungsbeispiele**

Zur Charakterisierung der katalytischen Eigenschaften wurden die beispielgemäßen Produkte nach der Überführung in die azide Wasserstofform in der Isomerisierung von m-Xylen im Laborimpulssystem unter nachfolgend aufgeführten Bedingungen eingesetzt:

Einwaage an granuliertem Pulver (0,315 - 0,45 mm): 50 mg

Reaktionstemperatur: 573 K

Einspritzmenge an m-Xylen: 5 µl

Folgende Selektivitäten (ausgedrückt als molares Verhältnis von p-Xylen zu o-Xylen im Reaktionsprodukt= wurden ermittelt:

| Probe gemäß Beispiel: | $Zd_i$ /pm | p-Xylen/o-Xylen |
|---|---|---|
| 1 | 974,82 | 1,9 |
| 2 | 972,90 | 3,6 |
| 3 | 973,30 | 2,8 |
| 4 | 974,20 | 2,1 |
| 5 | 973,90 | 2.5. |

**Patentansprüche**

1. Verfahren zur Herstellung synthetischer, kristalliner, metallhaltiger Borosilikate mit Pentasilstruktur in einem Kristallisationsprozeß aus einer Reaktionsmischung, die Verbindung von Silicium, Bor, Natrium, Hydroxidionen sowie Wasser enthält, gekennzeichnet dadurch, daß die Reaktionsmischung keine Struktur dirigierenden organischen Verbindungen oder Stickstoffverbindungen enthält und daß der Reaktionsmischung mindestens eine Aluminiumverbindung und mindestens ein Nebengruppenelement der 4. Periode des Periodensystems zugesetzt wird.

2. Verfahren gemäß Anspruch 1, gekennzeichnet dadurch, daß die Aluminiumverbindung aus Aluminiumoxid, deren hydratisierte Derivaten oder Aluminaten oder einem löslichen Aluminiumsalz besteht.

3. Verfahren gemäß Anspruch 1 und 2, gekennzeichnet dadurch, daß die zugesetzte Menge an Aluminium, bezogen auf den Feststoffanteil mindestens 0,5 jedoch nicht mehr als 10 prozentuale Massenanteile beträgt.

4. Verfahren nach Anspruch 1, dadurch gekennzeichnet, daß es sich bei den Elementen aus der 4. Periode des Periodensystems der Elemente um Chromium, Nickel, Kobalt, Eisen, Gallium, Zink und/oder Mangan handelt.

5. Verfahren nach Anspruch 1 und 2, dadurch gekennzeichnet, daß die Reaktionsmischung folgende Zusammensetzung, ausgedrückt in Molverhältnissen der Elemente bzw. deren Oxide, aufweist:
   $Na_2O/SiO_2$    0,05 bis 0,5 und
   $H_2O/SiO_2$    5 bis 150 beträgt.
   $SiO_2/Al_2O_3$    $\geqq$ 10
   B/(B+Al)    0,1 bis 1,0
   M/(Al+B)    0,05 bis 5
   M steht für ein Element der 4. Periode des Periodensystems der Elemente.

6. Verfahren gemäß einem oder mehrerer der vorhergehenden Ansprüche, welches über einen Zeitraum von 1 bis 100 Stunden unter hydrothermalen Bedingungen bei 403 K bis 593 K geführt wird.

7. Ein Verfahren gemäß Anspruch 5, in dem $SiO_2/Al_2O_3$ im Bereich von 30 bis 60 liegt.

8. Ein Verfahren gemäß Anspruch 5, in dem B/(B+Al) im Bereich von 0,15 bis 0,5 liegt.

9. Ein Verfahren gemäß Anspruch 5, in dem M/(B+Al) im Bereich von 0,1 bis 3 liegt.

**10.** Ein Verfahren gemäß Anspruch 5, in dem $Na_2/SiO_2$ im Bereich von 0,08 bis 0,2 liegt.

**11.** Ein Verfahren gemäß Anspruch 5, in dem $H_2O/SiO_2$ im Bereich von 20 bis 100 liegt.

**12.** Ein Verfahren nach Anspruch 6, welches über einen Zeitraum von 8 bis 40 Stunden betrieben wird.

**13.** Ein Verfahren gemäß Anspruch 6, welches bei einer Temperatur von 423 K bis 593 K betrieben wird.

**14.** Ein Verfahren gemäß einem oder mehreren der vorhergehenden Ansprüche bei dem die Reaktionsmischung vor der Kristallisation über einen Zeitraum von 1 bis 24 Stunden bei Raumtemperatur gealtert wird.

**15.** Verfahren gemäß einem der vorhergehenden Ansprüche, bei dem der Reaktionsmischung 0,5 bis 10 prozentuale Massenteile eines kristallinen oder teilkristallinen Borosilikaten gleicher oder ähnlicher Struktur zugesetzt werden.

**16.** Synthetisches, kristallines, metallhaltiges Borosilikat mit Pentasilstruktur das durch ein Verfahren gemäß einem der vorhergehenden Ansprüche erhalten wurde, gekennzeichnet durch folgende Merkmale:
0 8 bis 1,5 $Na_2O * x\,B_2O_3 * y\,Al_2O_3 * z\,M_2O * 20$ bis 60 $SiO_2$ mit x+y = 1, x/(x+y) = 0,1 bis 1,0 und z/(x+y)=0,05 bis 5, wobei M für die unter Anspruch 5 genannten Elemente steht, das frei von organischen Bestandteilen und Stickstoffverbindungen ist und die folgenden Netzebenenabstände besitzt :

| Netzebenenabstand (nm) | | relative Intensität |
|---|---|---|
| 1,11 | +/- 0,02 | st |
| 0,980 | +/- 0,02 | st |
| 0,587 | +/- 0,01 | s |
| 0,557 | +/- 0,01 | s |
| 0,548 | +/- 0,01 | ss |
| 0,421 | +/- 0,01 | s |
| 0,386 | +/- 0,007 | sst |
| 0,381 | +/- 0,007 | st |
| 0,372 | +/- 0,005 | m |
| 0,369 | +/- 0,005 | m |
| 0,362 | +/- 0,005 | m |
| 0,340 | +/- 0,005 | s |
| 0,329 | +/- 0,003 | ss |
| 0,302 | +/- 0,003 | s |
| 0,297 | +/- 0,003 | s |
| 0,294 | +/- 0,003 | s |

**17.** Katalysator, hergestellt unter Verwendung eines synthetischen, kristallinen, metallhaltigen Borosilikaten gemäß Anspruch 16.

**Claims**

**1.** Process for preparing, with the aid of a crystallisation method, metal containing synthetic crystalline borosilicates with pentasil structure from a reaction mixture containing a compound of silicon, boron, sodium, hydroxide ions and water, characterised in that the reaction mixture does not contain any structure-determining organic compounds or nitrogen compounds and that at least one aluminium compound and at least one sub-group element pertaining to the 4th period of the Periodic Table is added to the reaction

7

mixture.

2. Process according to Claim 1, characterised in that the aluminium compound consists of aluminium oxide, its hydrated derivatives or aluminates or a soluble aluminium salt.

3. Process according to Claims 1 and 2, characterised in that the quantity of aluminium added amounts, in relation to the share of solids to at least 0.5 but not more than 10 percent by weight.

4. Process according to Claim 1, characterised in that the elements pertaining to the 4th period of the Periodic Table of elements are chromium, nickel, cobalt, iron, gallium, zinc and/or manganese.

5. Process according to Claims 1 and 2, characterised in that the reaction mixture has the following composition expressed in molar ratios of the elements or their oxides respectively:

| | |
|---|---|
| $Na_2O/SiO_2$ | 0.05 to 0.5 and |
| $H_2O/SiO_2$ | 5 to 150. |
| $SiO_2/Al_2O_3$ | $\geqq 10$ |
| $B/(B+Al)$ | 0.1 to 1.0 |
| $M/(Al+B)$ | 0.05 to 5 |

M signifies an element pertaining to the 4th period of the Periodic Table of elements.

6. Process according to one or several of the preceding claims carried out during a time of 1 to 100 hours under hydrothermal conditions at 403 K to 593 K.

7. A process according to Claim 5, in which $SiO_2/Al_2O_3$ is within the range from 30 to 60.

8. A process according to Claim 5, in which $B/(B+Al)$ is within the range from 0.15 to 0.5.

9. A process according to Claim 5, in which $M/(B+Al)$ is within the range from 0.1 to 3.

10. A process according to Claim 5, in which $Na_2/SiO_2$ is within the range from 0.08 to 0.2.

11. A process according to Claim 5, in which $H_2O/SiO_2$ is within the range from 20 to 100.

12. A process according to Claim 6, which is carried out during a time of 8 to 40 hours.

13. A process according to Claim 6, which is carried out at a temperature of between 423 K and 593 K.

14. A process according to one or several of the preceding claims, in which the reaction mixture is aged at room temperature prior to crystallisation during a period between 1 and 24 hours.

15. Process according to one of the preceding claims, in which 0.5 to 10 percent by weight of a crystalline or partly crystalline borosilicate with identical or similar structure are added to the reaction mixture.

16. Synthetic, crystalline, metal containing borosilicate with pentasil structure, produced by a process according to one of the preceding claims and characterised by:

0.8 to 1.5 $Na_2O * x\ B_2O_3 * y\ Al_2O_3 * zM_2O * 20$ to $60\ SiO_2$ with $x+y = 1$, $x/(x+y) = 0.1$ to 1.0 and $z/(x+y) = 0.05$ to 5, where M signifies one of the elements listed in Claim 5, which is free of organic constituents and nitrogen compounds and exhibits the following interplanar distances:

**Annex A**

| Interplanar distance (nm) | relative intensity |
|---|---|
| 1.11 +/- 0.02 | h |
| 0.980 +/- 0.02 | h |
| 0.587 +/- 0.01 | l |
| 0.557 +/- 0.01 | l |
| 0.548 +/- 0.01 | vl |
| 0.421 +/- 0.01 | l |
| 0.386 +/- 0.007 | vh |
| 0.381 +/- 0.007 | h |
| 0.372 +/- 0.005 | m |
| 0.369 +/- 0.005 | m |
| 0.362 +/- 0.005 | m |
| 0.340 +/- 0.005 | l |
| 0.329 +/- 0.003 | vl |
| 0.302 +/- 0.003 | l |
| 0.297 +/- 0.003 | l |
| 0.294 +/- 0.003 | l |

17. Catalyst produced using a synthetic, crystalline metal containig borosilicate according to Claim 16.

**Revendications**

1. Procédé de préparation de borolisicates à structure pentasil synthétiques, cristallins contenant un métal dans un processus de cristallisation à partir d'un mélange réactionnel qui comprend des composés de silicium, bore, sodium, ions hydroxyde ainsi que l'eau, caractérisé en ce que le mélange réactionnel est sans composés organiques orientant la structure, ou composés de l'azote, et que le mélange réactionnel comprend au moins un composé d'aluminium et qu'on ajoute au moins un élément du sous-groupe de la 4ème période du système périodique.

2. Procédé selon la revendication 1 caractérisé en ce que le composé de l'aluminium est constitué d'oxyde d'aluminium, ses dérivés hydratés ou les aluminates ou d'un sel soluble d'aluminium.

3. Procédé selon la revendication 1 et 2, caractérisé en ce que la quantité ajoutée d'aluminium, rapportée à la teneur en solide représente au moins 0,5 pourcent en masse mais pas plus de 10 pourcents de la teneur en solide.

4. Procédé selon la revendication 1, caractérisé en ce que pour l'élément de la 4ème période du tableau périodique des éléments il s'agit du chrome, nickel, cobalt, fer, gallium, zinc et/ou manganèse.

5. Procédé selon la revendication 1 et 2, caractérisé en ce que le mélange réactionnel présente la composition suivante, exprimée en proportions molaires des éléments ou de leurs oxydes :
   $Na_2O/SiO_2$      0,05 à 0,5 et
   $H_2O/SiO_2$      5 à 150
   $SiO_2/Al_2O_3$      $\geqq$ 10
   B/(B+Al)      0,1 à 1,0
   M/(Al+B)      0,05 à 5
   M représente un élément de la 4ème période du tableau périodique des éléments.

6. Procédé selon l'une ou plusieurs des revendications précédentes, qui est conduit sur un intervalle de temps de 1 à 100 heures dans des conditions hydrothermales entre 403 K et 593 K.

7. Procédé selon la revendication 5, dans lequel $SiO_2/Al_2O_3$ est compris entre 30 et 60.

8. Procédé selon la revendication 5, dans lequel B/(B+Al) est compris entre 0,15 et 0,5.

9. Procédé selon la revendication 5, dans lequel M/(B+Al) est compris entre 0,1 et 3.

10. Procédé selon la revendication 5, dans lequel $Na_2O/SiO_2$ est compris entre 0,08 et 0,2.

11. Procédé selon la revendication 5, dans lequel $H_2O/SiO_2$ est compris entre 20 et 100.

12. Procédé selon la revendication 6, qu'on fait fonctionner sur un intervalle de temps de 8 à 40 heures.

13. Procédé selon la revendication 6, qu'on fait fonctionner à une température comprise entre 423 K et 593 K.

14. Procédé selon l'une ou plusieurs des revendications précédentes dans lequel on laisse mûrir le mélange réactionnel avant la cristallisation sur un intervalle de temps de 1 à 24 heures à température ambiante.

15. Procédé selon l'une des revendications précédentes, selon lequel 0,5 à 10 % en masse d'un borosilicate cristallin ou en partie cristallin de structure identique ou similaire sont ajoutés au mélange réactionnel.

16. Borosilicate à structure pentasil synthétique cristallin contenant un méthane obtenu par un procédé selon l'une des revendications précédentes, caractérisé par les caractéristiques suivantes :
0,8 à 1,5 $Na_2O$ * x $B_2O_3$ * y $Al_2O_3$ * z $M_2O$ * 20 à 60 $SiO_2$ avec x+y = 1, x/(x+y) = 0.1 à 1.0 et z/(x+y) = 0.05 à 5, où M représente l'élément indiqué à la revendication 5, en ce qu'il est exempt de composants organiques et de composés azotés et possède les paramètres de réseau :

| Paramètres de réseau (nm) | | Intensité relative |
|---|---|---|
| 1,11 | +/- 0,02 | h |
| 0,980 | +/- 0,02 | h |
| 0,587 | +/- 0,01 | b |
| 0,557 | +/- 0,01 | b |
| 0,548 | +/- 0,01 | tb |
| 0,421 | +/- 0,01 | b |
| 0,386 | +/- 0,007 | th |
| 0,381 | +/- 0,007 | h |
| 0,372 | +/- 0,005 | m |
| 0,369 | +/- 0,005 | m |
| 0,362 | +/- 0,005 | m |
| 0,340 | +/- 0,005 | b |
| 0,329 | +/- 0,003 | tb |
| 0,302 | +/- 0,003 | b |
| 0,297 | +/- 0,003 | b |
| 0,294 | +/- 0,003 | b |

17. Catalyseur fabriqué par utilisation d'un borosilicate synthétique cristallin contenant un métal.